# FASCICULE DE BREVET EUROPEEN

(11) **EP 4 027 860 B1**
(45) Date de publication et mention de la délivrance du brevet: **21.05.2025**
(21) Numéro de dépôt: 20781046.6
(22) Date de dépôt: 11.09.2020
(51) Int. Cl.: A61B 3/00, A61B 3/024, A61B 3/13, A61B 3/16, A61B 3/18

(54) **DISPOSITIF DE MESURE OPHTALMOLOGIQUE POLYVALENT**
OPHTHALMOLOGISCHES MEHRZWECKMESSGERÄT
MULTIPURPOSE OPHTHALMOLOGICAL MEASURING DEVICE

(30) Priorité: 13.09.2019 FR 1910131
(43) Date de publication de la demande: 20.07.2022
(73) Titulaire: E-Swin Developpement, 78550 Houdan (FR)
(72) Inventeur: BROTTIER, Yves-Vincent, 78113 ADAINVILLE (FR); OBIN, Arnaud, 78660 PARAY-DOUAVILLE (FR); PERRIN, Nelson, 78730 SAINTE-MESME (FR)
(74) Mandataire: Plasseraud IP
(86) Numéro de dépôt international: PCT/FR2020/051578
(87) Numéro de publication internationale: WO 2021/048509

(56) Documents cités:
- EP-A1- 1 854 400
- EP-A2- 0 815 789
- WO-A1-2015/087786
- AT-B- 317 410
- JP-A- H03 173 527
- US-A1- 2017 285 337

## Description

### Domaine technique

L'invention relève du domaine des dispositifs ophtalmologiques permettant de faire des mesures sur les yeux de patients.

### Technique antérieure

Des dispositifs ophtalmologiques pour lesquels les patients doivent regarder un point particulier pendant qu'un praticien observe leurs yeux sont connus. Des exemples de dispositifs ophtalmiques sont décrits dans les documents EP 0 815 789 A2, JP H03 173527 A et US 2017/285337 A1.

### Problème technique

A ce jour ces dispositifs ophtalmologiques n'offrent pas une grande polyvalence.

### Exposé de l'invention

La présente demande concerne un dispositif de mesure ophtalmologique polyvalent en ce qu'il regroupe plusieurs types de dispositifs de mesure et est utilisable selon plusieurs configurations.

Plus précisément la présente demande propose un dispositif de mesure ophtalmologique qui comporte un boîtier de mesures oculaires comprenant une face avant pourvue d'un cadre entourant une plaque translucide de dimension et de courbure adaptées pour recouvrir le champ oculaire dudit patient, un dispositif d'éclairage de la plaque translucide derrière la plaque translucide, au moins une caméra munie d'un objectif disposé en correspondance d'un trou dans la plaque translucide, une ou plusieurs sources de lumière ponctuelles dans des ouvertures sur la plaque translucide pour illuminer les yeux du patient et des moyens de fixation d'un ou plusieurs dispositifs complémentaires de mesure ophtalmologiques reçus sur la plaque translucide.

L'invention propose ainsi un dispositif compact muni de plusieurs moyens de mesure pour réaliser plusieurs examens différents.

Préférablement, le dispositif d'éclairage de la plaque translucide derrière la plaque translucide comporte une boîte d'intégration diffusant la lumière d'une source de lumière blanche et/ou infrarouge proche.

L'éclairage est ainsi homogène au niveau de la plaque translucide.

Selon un mode de réalisation avantageux, les sources de lumière ponctuelles comprennent des diodes électroluminescentes bleues.

De telles diodes sont adaptées à des examens sous fluorescéine.

Selon un mode de réalisation particulier, le dispositif de mesure ophtalmologique de la demande est équipé d'un dispositif complémentaire d'examen constitué d'un cadre translucide porteur d'une mire pourvue d'au moins un trou aligné avec un dit objectif de caméra, ledit cadre comportant des moyens de fixation complémentaires des moyens de fixation dudit dispositif de mesure ophtalmologique.

Selon un premier mode de réalisation, le boîtier comporte des moyens de fixation sur un casque se positionnant sur la tête d'un patient. Les moyens de fixation peuvent notamment être disposés sous un second capot supérieur du dispositif et destinés à coopérer avec des moyens de fixation complémentaires du casque.

Selon un second mode de réalisation alternatif ou complémentaire, le boîtier comporte des moyens de fixation sur un bâti ophtalmologique de table sur lequel le patient vient s'appuyer.

Les moyens de fixation du dispositif sur ledit bâti ophtalmologique de table peuvent comprendre un capot supérieur pourvu de logements de réceptions de terminaisons de montants du bâti.

Selon un mode de réalisation avantageux, le boîtier comporte au moins une carte électronique à microprocesseur comportant des moyens de pilotage des moyens d'éclairage et des caméras, des moyens d'acquisition des données vidéo ou photographiques issus des caméras et des moyens de calcul comprenant un ou plusieurs programmes informatiques de mesure comprenant des traitements informatiques des données vidéo ou photographiques acquises, un écran tactile intégré au dispositif pour piloter le dispositif selon le ou les programmes informatiques de mesures et visualiser des résultats de mesures ophtalmiques issus des traitements informatiques réalisés par la carte électronique.

Le dispositif de l'invention réalise ainsi un appareil d'examen autonome et polyvalent.

L'invention propose en outre selon un premier mode de réalisation, un ensemble de mesure ophtalmologique comportant un casque de mesures ophtalmologiques se positionnant sur la tête d'un patient et un dispositif de mesure ophtalmologique fixé au casque.

L'invention propose aussi, selon un second mode de réalisation, un ensemble de mesure ophtalmologique comportant un bâti ophtalmologique de table et un dispositif de mesure ophtalmologique fixé audit bâti.

Le dispositif de mesure de l'invention est ainsi utilisable dans deux configurations moyennant le changement d'une platine d'interface.

### Brève description des dessins

D'autres caractéristiques, détails et avantages de l'invention apparaîtront à la lecture de la description détaillée ci-après, et à l'analyse des dessins annexés, sur lesquels :
[Fig. 1] montre une vue en perspective d'un premier mode de réalisation d'un ensemble de mesure ophtalmologique de l'invention;
[Fig. 2] montre un détail de la figure 1;
[Fig. 3] est une vue schématique d'une partie du dispositif de l'invention;
[Fig. 4] montre une vue en perspective d'un second mode de réalisation d'un ensemble de mesure ophtalmologique de l'invention;
[Fig. 5] montre une vue en perspective d'un exemple de réalisation du dispositif de l'invention en rapport avec la figure 4;
[Fig. 6] représente un cadre translucide porteur d'une mire utilisable dans le cadre d'un mode de réalisation de l'invention.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

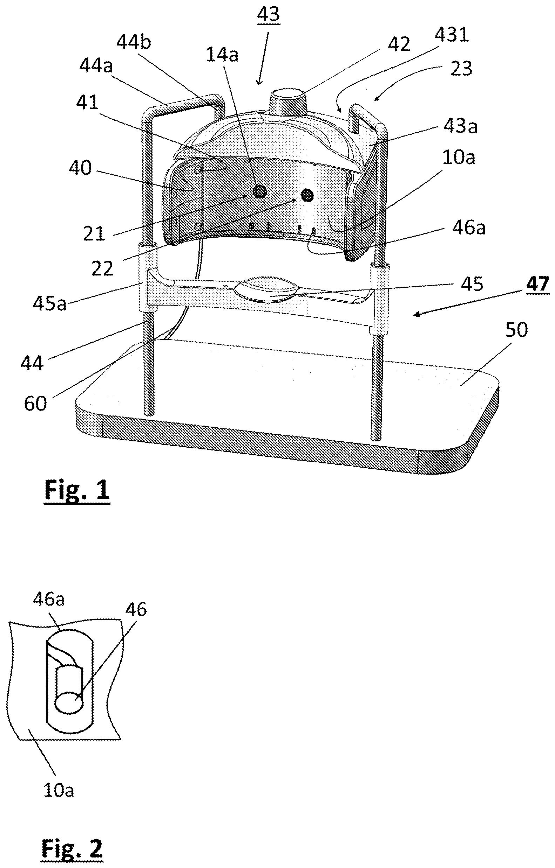

Il est maintenant fait référence à la figure 1. Sur cette figure correspondant à un premier mode de réalisation d'un ensemble de mesure ophtalmologique de l'invention, un dispositif de mesure ophtalmologique 43 est monté sur un bâti ophtalmologique de de table 47.

Le bâti comporte des montants 44, un support de menton 45, un plateau support 50. Les montants 44 ont leur extrémité inférieure fixées sur le plateau support 50, une partie verticale recevant des bras 45a de fixation du support de menton 45 et une partie supérieure courbée 44a pourvue de terminaisons 44b tournées vers le bas et s'insérant dans des logements 431 sur un capot supérieur 43a du dispositif de mesure ophtalmologique par exemple vissé sur le boîtier 43.

La fixation des montants sur le dispositif peut être réalisée par différents moyens traditionnels comme des vis solidarisant les terminaisons des montants dans les logements dudit capot.

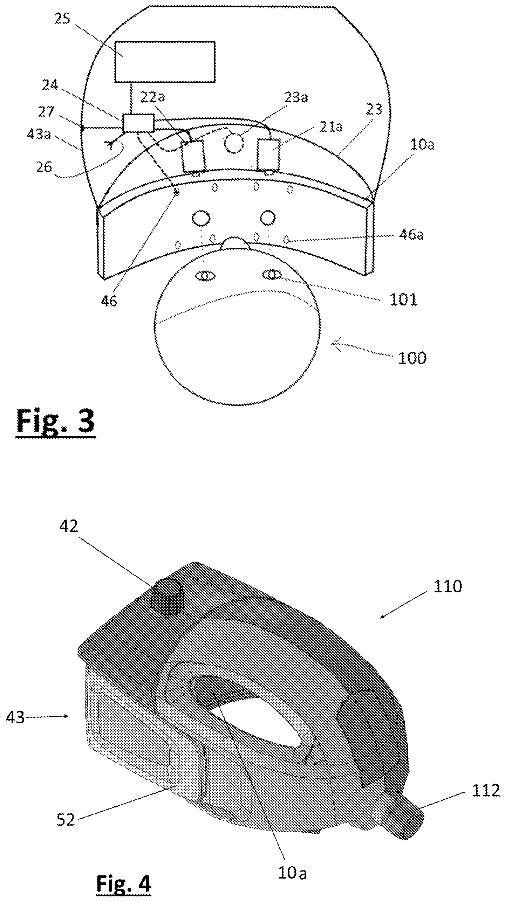

Le dispositif de mesure ophtalmologique comporte un boîtier 43 qui va contenir des moyens permettant de réaliser plusieurs types de mesures ophtalmologiques. Ces moyens sont constitués notamment de dispositifs d'éclairement des yeux. Le dispositif comporte un moyen d'éclairement diffus constitué par une plaque translucide 10a courbe derrière laquelle se trouve un dispositif de rétroéclairage homogène 23 tel qu'une boîte d'intégration schématisée en figure 3 enfermée à l'arrière du boîtier 43 et des moyens d'éclairage ponctuels 46 représentés en figure 2 et répartis ici sur un bord supérieur et un bord inférieur de la plaque translucide dans des ouvertures 46a.

Le dispositif de rétroéclairage peut comporter une ou plusieurs LEDs 23a en lumière blanche, en lumière infrarouge proche ou d'autres longueurs d'ondes selon les examens ophtalmologiques à réaliser.

Les moyens d'éclairage ponctuels sont notamment des diodes électroluminescentes (LEDs) et comprennent des LEDs bleues adaptées pour des examens avec fluorescéine.

Les moyens d'éclairage sont mis en service et éteints indépendamment au moyen d'une carte électronique 24 dans le boîtier.

Un autre élément important du dispositif est la présence d'au moins une caméra et préférablement deux caméras pour permettre des examens sur les deux yeux 101 d'un patient 100. Selon l'exemple des figures 1 et 3, deux caméras 21a, 22a sont utilisées. Les caméras ont leurs objectifs 21, 22 disposés dans des trous 14a réalisés dans une partie médiane de la plaque translucide 10a. Ces caméras sont positionnées de sorte que leurs objectifs soient en face des yeux 101 d'une majorité de patients pour pouvoir les filmer ou les photographier.

Les caméras sont des caméras miniatures, par exemple des caméras à capteurs CCD 1/4" d'une définition de 1920x1080 dont les objectifs sont compatibles avec des trous 14a de de 10mm à 15mm.

Le dispositif est autonome et le boitier schématisé en figure 3 comporte pour ce faire au moins une carte électronique à microprocesseur 24. Cette carte comporte selon l'exemple:
- des moyens de pilotage des moyens d'éclairage 23, 46 et des caméras 21, 22,
- des moyens d'acquisition des données vidéo ou photographiques issus des caméras,
- des moyens de calcul adaptés à procéder à des traitements informatiques des données vidéo ou photographiques acquises.
- un écran tactile 25 intégré au dispositif et relié à la carte électronique pour permettre à un opérateur de piloter le dispositif et visualiser des résultats de mesures ophtalmiques issus des traitements informatiques des données issues des caméras réalisés par la carte électronique.

La carte électronique à microprocesseur (24) comporte de manière connue un processeur, de la mémoire vive, de la mémoire de stockage permanent avec une partie de stockage d'un programme de gestion du dispositif, une partie de stockage d'un programme de mesure et une partie de stockage de données de mesures des patients,

Les programmes de mesure intégrés à la carte électronique sont des programmes de réalisation de mesures ophtalmiques utilisant les divers moyens d'éclairage et les caméras pour faire des examens oculaires sur les patients comme des examens de la cornée, des examens de rupture de film oculaire ou autres examens utilisant les moyens d'éclairage et les caméras.

Selon l'exemple la carte électronique comporte un module Wi-Fi avec une antenne 26 permettant de communiquer avec un appareil tel qu'une tablette informatique par exemple programmée pour fournir une recopie des informations présentes sur l'écran 25 sont présentes. La carte comporte en outre un port informatique 27 tel qu'un port USB qui permet par exemple de réaliser des sauvegardes des mesures sur un système informatique distant ou des mises à jour du ou des programmes de la carte 24.

Dans la configuration casque, le boîtier peut fonctionner sur une batterie embarquée et dans la configuration sur bâti, le boîtier peut être relié à une alimentation externe.

La figure 4 représente un second mode de réalisation d'un ensemble de mesure ophtalmologique de l'invention. Selon ce mode de réalisation, le dispositif de mesure ophtalmologique 43 est fixé sur un casque 110 destiné à recouvrir la tête du patient ce qui procure un meilleur suivi des yeux du patient par les caméras. Le casque possède une molette 112 actionnant un mécanisme de serrage de la tête du patient. Le dispositif ophtalmologique est fixé sur le casque par des moyens d'accrochage 51 situés sous un capot supérieur 43b du boîtier, ces moyens comportant des pattes 51a s'insérant dans des rainures complémentaires du casque pour solidariser le dispositif au casque.

Comme le capot 43a, le capot 43b peut être vissé sur le boîtier.

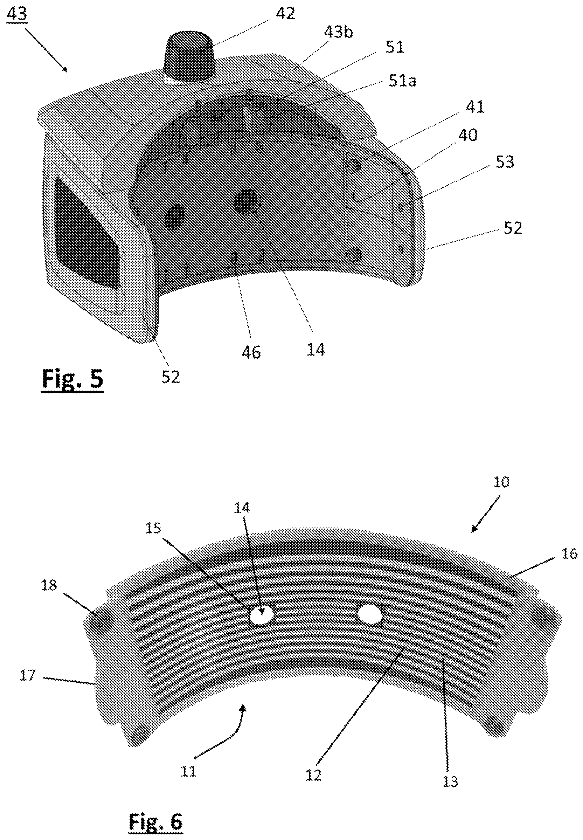

Selon la figure 5, le dispositif comporte des moyens 41 de fixation d'un support d'un dispositif complémentaire d'examen. Le support, dans l'exemple représenté à la figure 6, est un cadre ou une plaque translucide amovible 16 porteur d'une mire 10 pourvue d'au moins un trou 14 aligné avec un dit objectif de caméra, ledit cadre comportant des moyens de fixation 18 complémentaires des moyens de fixation 41. Les moyens de fixation 18, 41 peuvent être des moyens de fixation tels que des aimants ou des fixations de type à boutons pression. Le support comporte des oreilles de préhension 17 qui permettent de le positionner et de le retirer aisément selon l'examen qui doit être pratiqué.

Le dispositif de mesure ophtalmologique de l'invention peut donc être fabriqué selon deux configurations, une configuration d'appareil de table et une configuration sur casque à partir d'un boîtier standardisé en utilisant une platine d'interface adéquate pour le montage sur bâti ou sur casque.

Le dispositif de mesure de l'invention qui utilise des matériaux légers compatibles d'une utilisation sur casque est ainsi un dispositif modulaire qui regroupe plusieurs systèmes d'examen. Ce dispositif objet de revendications qui suivent n'est pas limité aux exemples représentés et, par exemple, les sources d'éclairage ponctuelles représentées sur le bord de la plaque translucide peuvent aussi être positionnés autour des trous de passage des caméras ou ailleurs.

## Revendications

1. Dispositif de mesure ophtalmologique comportant un boîtier (43) de mesures oculaires comprenant une face avant (40) pourvue d'un cadre entourant une plaque translucide (10a) de dimension et de courbure adaptées pour recouvrir le champ oculaire dudit patient, un dispositif d'éclairage (23) de la plaque translucide derrière la plaque translucide, au moins une caméra (21, 22) munie d'un objectif disposé en correspondance d'un trou (14) dans la plaque translucide, une ou plusieurs sources de lumière ponctuelles (46) dans des ouvertures (46a) sur la plaque translucide pour illuminer les yeux du patient et des moyens de fixation (41) d'un ou plusieurs dispositifs complémentaires de mesure ophtalmologiques reçus sur la plaque translucide.

2. Dispositif de mesure ophtalmologique selon la revendication 1, pour lequel le dispositif d'éclairage (23) de la plaque translucide est un dispositif de rétroéclairage homogène de ladite plaque.

3. Dispositif de mesure ophtalmologique selon la revendication 1 ou 2, pour lequel le dispositif d'éclairage (23) de la plaque translucide derrière la plaque translucide comporte une boîte d'intégration diffusant la lumière d'une source de lumière blanche et/ou infrarouge proche.

4. Dispositif de mesure ophtalmologique selon la revendication 1, 2 ou 3, pour lequel les sources de lumière ponctuelles (46) comprennent des diodes électroluminescentes bleues.

5. Dispositif de mesure ophtalmologique selon l'une quelconque des revendications précédentes, équipé d'un dispositif complémentaire d'examen constitué d'un cadre translucide (16) porteur d'une mire (10) pourvue d'au moins un trou (14) aligné avec un dit objectif de caméra, ledit cadre comportant des moyens de fixation (18) complémentaires des moyens de fixation (41) dudit dispositif de mesure ophtalmologique.

6. Dispositif de mesure ophtalmologique selon l'une quelconque des revendications précédentes, pour lequel le boîtier comporte des moyens de fixation (51) sur un casque (110) se positionnant sur la tête d'un patient.

7. Dispositif de mesure ophtalmologique selon la revendication 6 pour lequel lesdits moyens de fixation sont disposés sous un capot supérieur (43b) du dispositif et destinés à coopérer avec des moyens de fixation complémentaires du casque.

8. Dispositif de mesure ophtalmologique selon l'une quelconque des revendications précédentes, pour lequel le boîtier comporte des moyens de fixation sur un bâti ophtalmologique de table (47) sur lequel le patient vient s'appuyer.

9. Dispositif de mesure ophtalmologique selon la revendication 8, pour lequel les moyens de fixation sur ledit bâti ophtalmologique de table (47) comprennent un capot supérieur (43a) pourvu de logements (431) de réceptions de terminaisons (44b) de montants du bâti.

10. Dispositif de mesure ophtalmologique selon l'une quelconque des revendications précédentes, pour lequel le boîtier est un boitier autonome qui comporte au moins une carte électronique à microprocesseur (24) comportant des moyens de pilotage des moyens d'éclairage et des caméras, des moyens d'acquisition des données vidéo ou photographiques issus des caméras et des moyens de calcul comprenant un ou plusieurs programmes informatiques de mesure comprenant des traitements informatiques des données vidéo ou photographiques acquises, un écran tactile (25) intégré au dispositif pour piloter le dispositif selon le ou les programmes de mesures et visualiser des résultats de mesures ophtalmiques issus des traitements informatiques réalisés par la carte électronique (24).

11. Ensemble de mesure ophtalmologique comportant un casque (110) de mesures ophtalmologiques se positionnant sur la tête d'un patient et un dispositif de mesure ophtalmologique (43) selon l'une quelconque des revendications précédentes fixé au casque.

12. Ensemble de mesure ophtalmologique comportant un bâti ophtalmologique de table (47) et un dispositif de mesure ophtalmologique selon l'une quelconque des revendications 1 à 10 fixé audit bâti.

## Patentansprüche

1. Ophthalmologische Messvorrichtung, umfassend ein Gehäuse (43) für Augenmessungen, das eine Vorderseite (40) umfasst, die mit einem Rahmen versehen ist, der eine lichtdurchlässige Platte (10a) umgibt, deren Abmessungen und Krümmung dazu angepasst sind, das Augenfeld des Patienten abzudecken, eine Beleuchtungsvorrichtung (23) für die lichtdurchlässige Platte hinter der lichtdurchlässigen Platte, wenigstens eine Kamera (21, 22) mit einem Objektiv, das entsprechend einem Loch (14) in der lichtdurchlässigen Platte angeordnet ist, eine oder mehrere punktförmige Lichtquellen (46) in Öffnungen (46a) auf der lichtdurchlässigen Platte zur Beleuchtung der Augen des Patienten und Befestigungsmittel (41) einer oder mehrerer ergänzender ophthalmologischer Messvorrichtungen, die auf der lichtdurchlässigen Platte aufgenommen sind.

2. Ophthalmologische Messvorrichtung nach Anspruch 1, wobei die Beleuchtungsvorrichtung (23) der lichtdurchlässigen Platte eine Vorrichtung zur homogenen Hintergrundbeleuchtung der Platte ist.

3. Ophthalmologische Messvorrichtung nach Anspruch 1 oder 2, wobei die Beleuchtungsvorrichtung (23) der lichtdurchlässigen Platte hinter der lichtdurchlässigen Platte eine Integrationsbox umfasst, die das Licht einer Weißlichtquelle und/oder einer Nahinfrarotlichtquelle streut.

4. Ophthalmologische Messvorrichtung nach Anspruch 1, 2 oder 3, wobei die Punktlichtquellen (46) blaue Leuchtdioden umfassen.

5. Ophthalmologische Messvorrichtung nach einem der vorhergehenden Ansprüche, ausgestattet mit einer ergänzenden Untersuchungsvorrichtung, die aus einem lichtdurchlässigen Rahmen (16) besteht, der ein Visier (10) trägt, das mit wenigstens einem Loch (14) versehen ist, das mit einem Kameraobjektiv ausgerichtet ist, wobei der Rahmen Befestigungsmittel (18) aufweist, die zu den Befestigungsmitteln (41) der ophthalmologischen Messvorrichtung komplementär sind.

6. Ophthalmologische Messvorrichtung nach einem der vorhergehenden Ansprüche, wobei das Gehäuse Befestigungsmittel (51) an einem am Kopf eines Patienten positionierbaren Helm (110) aufweist.

7. Ophthalmologische Messvorrichtung nach Anspruch 6, wobei die Befestigungsmittel unter einer oberen Abdeckung (43b) der Vorrichtung angeordnet und dazu bestimmt sind, mit komplementären Befestigungsmitteln des Helms zusammenzuwirken.

8. Ophthalmologische Messvorrichtung nach einem der vorhergehenden Ansprüche, wobei das Gehäuse Befestigungsmittel an einem ophthalmologischen Tischgestell (47) aufweist, auf das sich der Patient stützt.

9. Ophthalmologische Messvorrichtung nach Anspruch 8, wobei die Befestigungsmittel an dem ophthalmologischen Tischgestell (47) eine obere Abdeckung (43a) umfassen, die mit Aufnahmen (431) zur Aufnahme von Endstücken (44b) von Gestellpfosten versehen ist.

10. Ophthalmologische Messvorrichtung nach einem der vorhergehenden Ansprüche, wobei das Gehäuse ein autonomes Gehäuse ist, das wenigstens eine elektronische Mikroprozessorkarte (24) umfasst, mit Mitteln zur Steuerung der Beleuchtungsmittel und Kameras, Mitteln zur Erfassung von Video- oder Fotodaten, die von den Kameras stammen, und Rechenmitteln, die ein oder mehrere Messcomputerprogramme umfassen, die die Verarbeitung der erfassten Video- oder Fotodaten umfassen, einen in die Vorrichtung integrierten Touchscreen (25) zur Steuerung der Vorrichtung nach dem oder den Messprogrammen und zur Visualisierung der Ergebnisse der Augenmessungen, die aus den von der elektronischen Karte (24) durchgeführten Computerverarbeitungen stammen.

11. Ophtalmologische Messanordnung mit einem Augenmesshelm (110), der am Kopf eines Patienten positionierbar ist, und einer an dem Helm befestigten ophtalmologischen Messvorrichtung (43) nach einem der vorhergehenden Ansprüche.

12. Ophthalmologische Messanordnung, umfassend ein ophthalmologisches Tischgestell (47) und eine am Gestell befestigte ophthalmologische Messvorrichtung nach einem der Ansprüche 1 bis 10.

## Claims

1. An ophthalmological measuring device, having an ocular measurement housing (43) comprising a front face (40) provided with a frame surrounding a translucent plate (10a) of a size and curvature suitable for covering the ocular field of said patient, a device (23) for lighting the translucent plate from behind the translucent plate, at least one camera (21, 22) provided with a lens placed in line with a hole (14) in the translucent plate, one or more point light sources (46) in openings (46a) on the translucent plate, for illuminating the eyes of the patient, and means (41) for fixing one or more complementary ophthalmological measuring devices received on the translucent plate.

2. The ophthalmological measuring device as claimed in claim 1, in which the device (23) for lighting the translucent plate is an homogeneous backlighting device for said plate.

3. The ophthalmological measuring device as claimed in claim 1 or 2, in which the device (23) for lighting the translucent plate from behind the translucent plate comprises an integration box diffusing the light from a white and/or near infrared light source.

4. The ophthalmological measuring device as claimed in claim 1, 2 or 3, in which the point light sources (46) comprise blue light emitting diodes.

5. The ophthalmological measuring device as claimed in any one of the preceding claims, equipped with a complementary examination device consisting of a translucent frame (16) carrying a test pattern (10) provided with at least one hole (14) aligned with a said camera lens, said frame comprising fixing means (18) complementing the fixing means (41) of said ophthalmological measuring device.

6. The ophthalmological measuring device as claimed in any one of the preceding claims, in which the housing comprises means (51) for fixing to a helmet (110) positioned on the head of a patient.

7. The ophthalmological measuring device as claimed in claim 6, in which said fixing means are arranged under an upper cover (43b) of the device and are intended to cooperate with complementary fixing means of the helmet.

8. The ophthalmological measuring device as claimed in any one of the preceding claims, in which the housing comprises means for fixing to an ophthalmological table frame (47) on which the patient leans.

9. The ophthalmological measuring device as claimed in claim 8, in which the means for fixing to said ophthalmological table frame (47) comprise an upper cover (43a) provided with seats (431) for receiving endings (44b) of uprights of the frame.

10. The ophthalmological measuring device as claimed in any one of the preceding claims, in which the housing is a stand-alone housing which comprises at least one electronic board with microprocessor (24) comprising means for controlling the lighting means and the cameras, means for acquiring video or photographic data coming from the cameras, and calculation means comprising one or more measurement computer programs comprising computer processing of the acquired video or photographic data, a touch screen (25) integrated into the device in order to control the device according to the measurement program(s) and to display results of ophthalmic measurements from the computer processing carried out by the electronic board (24).

11. An ophthalmological measuring assembly comprising an ophthalmological measuring helmet (110), positioned on the head of a patient, and an ophthalmological measuring device (43), as claimed in any one of the preceding claims, fixed to the helmet.

12. An ophthalmological measuring assembly comprising an ophthalmological table frame (47) and an ophthalmological measuring device, as claimed in any one of claims 1 through 10, fixed to said frame.
